# EUROPÄISCHE PATENTANMELDUNG

(11) **EP 1 854 399 A1**
(43) Veröffentlichungstag der Anmeldung: **14.11.2007**
(21) Anmeldenummer: 06009710.2
(22) Anmeldetag: 11.05.2006
(51) Int. Cl.: A61B 1/07, A61B 1/04, A61B 18/20

(54) **Endoskopisches oder minimalinvasives Instrument**

(71) Anmelder: GfE Medizintechnik GmbH, 90431 Nürnberg (DE)
(72) Erfinder: Zimmermann, Hanngörg, 91327 Gössweinstein (DE); Heinlein, Markus, Dr., 91327 Gössweinstein (DE)
(74) Vertreter: Hübner, Gerd

(57) **Zusammenfassung**

Ein endoskopisches oder minimalinvasives Instrument umfasst
- einen Kopf (1) zur Handhabung des Instruments und zum Anschluss externer Komponenten,
- eine starre oder flexible Röhre (4) am Kopf (1) zum Einführen in den zu untersuchenden Körper,
- ein optisches Betrachtungssystem (5) im Rohr (4) zur optischen Erfassung der zu untersuchenden Körperpartie,
- einem Lichtleitsystem (3) in der Röhre (4) zur Beleuchtung der zu untersuchenden Körperpartie, und
- eine lasergestützte Lichtzeigereinrichtung (7) zur Erzeugung eines Leuchtpunktes in der zu untersuchenden oder zu behandelnden Körperpartie.

## Beschreibung

Die Erfindung betrifft ein endoskopisches oder minimalinvasives Instrument mit den im Oberbegriff des Anspruches 1 angegebenen Merkmalen.

Eine gängige und zunehmend praktizierte Methode in der modernen Chirurgie ist die minimalinvasive Operationsmethode mit laparoskopischen Instrumenten. Hierbei werden entgegen der offenen Operationsmethode über kleine Öffnungen der Körperdecke oder über Körperöffnungen selbst Instrumente in das Körperinnere eingebracht. Neben den Instrumenten wird zur Visualisierung des Operationsfeldes eine Optik in Form eines Endoskops eingesetzt, die das Körperinnere vorzugsweise in Verbindung mit einem Kamerasystem auf einen Monitor abbildet.

Endoskope und minimalinvasive Instrumente sind in vielerlei Ausführungsformen je nach ihrem medizinischen Zweck aus dem Stand der Technik bekannt. Ihre Grundelemente umfassen einen Kopf zur Handhabung des Endoskops, zum Anschluss externer Komponenten, wie einer Lichtquelle zur Beleuchtung der zu untersuchenden Körperpartie und einem Okular bzw. eine CCD-Kamera zur Übertragung des Endoskop-Bildes auf einen Monitor, oder zur Halterung von Betätigungselementen des Instruments.

Ferner umfasst das Instrument eine starre oder flexible Röhre am Kopf, die in den zu untersuchenden Körper eingeführt wird. Im Rohr ist bei einem Endoskop beispielsweise ein optisches Betrachtungssystem zur visuellen Erfassung der zu untersuchenden Körperpartie vorhanden, das in der Regel aus einer Linse am distalen Rohrende und einem durch das Rohr führenden Glasfaser-Lichtleiter besteht, der im Kopf in dem erwähnten Okular oder CCD-Sensor der Kamera endet. Schließlich kann zur Beleuchtung der zu untersuchenden Körperpartie in das Rohr ein Lichtleitsystem auf der Basis von Glasfasern eingebaut sein, das das von der Lichtquelle am Kopf eingespeiste Licht zum Austritt am distalen Ende der Röhre leitet.

Üblicherweise setzt sich ein OP-Team bei einer minimalinvasiven Operation aus zwei Chirurgen zusammen. Der Operateur bedient hierbei die Instrumente und führt den Eingriff durch. Der Assistent hingegen ist für die Führung der Optik und eine ausreichende Darstellung des Operationsfeldes erforderlich. Somit ist es dem Assistenten nicht möglich, dem Operateur Auffälligkeiten direkt zu zeigen. Vielmehr muss er durch Richtungsangaben bzw. Beschreibungen dem Operateur Hinweise für die Durchführung geben.

Bei der Ausbildung von Chirurgen führt ferner der erfahrene Arzt die Optik und der auszubildende Chirurg bedient die Instrumente. Somit gibt es bei Komplikationen oder aber auch bei Anweisungen zum Operationsverlauf für den erfahrenen Chirurgen nur die Möglichkeit, die Instrumente zu übernehmen oder über mündliche Angaben die Anatomie oder den zu behandelnden Bereich zu deklarieren.

Der Erfindung liegt vor dem Hintergrund der geschilderten Problematik die Aufgabe zugrunde, ein endoskopisches oder minimalinvasives Instrument so weiterzubilden, dass kritische Stellen zu untersuchender oder zu operierender Körperpartien über ein Betrachtungssystem eindeutig kenntlich machbar sind.

Diese Aufgabe wird durch die im Kennzeichnungsteil des Anspruches 1 angegebenen Merkmale gelöst. Demnach ist eine lasergestützte Lichtzeigereinrichtung zur Erzeugung eines Leuchtpunktes in der zu untersuchenden Körperpartie in das Instrument integriert.

Bei Bedarf kann also auf Knopfdruck durch den kameraführenden Chirurgen ein Leuchtpunkt im OP-Feld erzeugt werden, wodurch die jeweilig betroffene Stelle eindeutig markiert wird.

Neben der normalen OP-Situation ist es damit auch möglich, im Rahmen von Lehrveranstaltungen bzw. bei Operationen in der Telemedizin und Telematik Auffälligkeiten eindeutig zu kennzeichnen.

Neben einer Anwendung des Laserpointers innerhalb eines bildgebenden Endoskops ist es auch möglich, minimalinvasive Instrumente mit einem Lichtleiter und Laserpointer auszustatten. Somit ergibt sich auch für den operierenden Chirurgen die Möglichkeit, eine Zielanpeilung und Kennzeichnung von zu lokalisierenden Stellen berührungslos von außen durchzuführen.

Neben der medizinischen Anwendung der Erfindung ist der Einsatz des erfindungsgemäßen Instruments auch in allen anderen, nichtmedizinischen Bereichen möglich, wo der Einsatz von Endoskopen erfolgt.

Bevorzugte Ausführungsformen des erfindungsgemäßen Instruments sind in den Unteransprüchen angegeben, deren Merkmale, Einzelheiten und Vorteile in der nachfolgenden Beschreibung anhand der beigefügten Zeichnungen näher erläutert werden. Es zeigen:
- Fig. 1: einen ausschnittsweisen Längsschnitt durch ein Endoskop,
- Fig. 2: eine weggeschnittene Perspektivdarstellung der Röhre des Endoskops gemäß Fig. 1 in einer erster Ausführungsform, und
- Fig. 3: eine weggeschnittene Perspektivdarstellung einer Endoskopröhre in einer zweiten Ausführungsform.

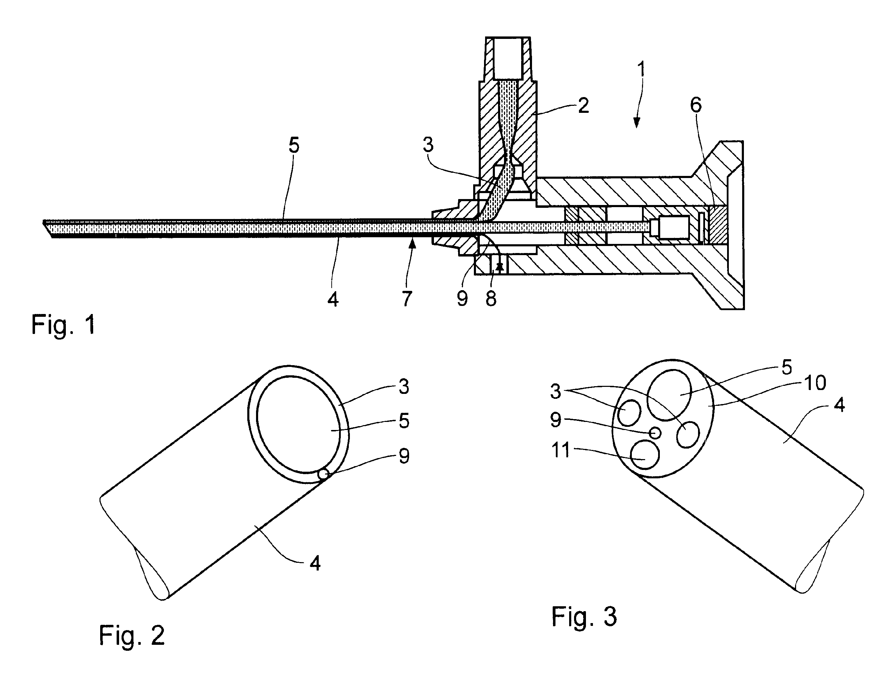

Das in Fig. 1 bis auf das in den Körper eingeführte distale Ende dargestellte Endoskop weist einen griffähnlichen Kopf 1 auf, an dem seitlich ein Anschlussstutzen 2 für eine nicht näher dargestellte Lichtquelle für das Lichtleitsystem 3 im Endoskop eingesetzt ist. Der Kopf 1 setzt sich in einer flexiblen oder - wie in den beigefügten Zeichnungen dargestellt - starren Röhre 4 fort, die mit ihrem (nicht gezeigten) distalen Ende in den Körper über eine Körperhöhlung oder ein Trokar in der Bauchdecke eingeführt und zu einer zu untersuchenden Körperpartie vorgeschoben wird. In der Röhre 4 ist ein optisches System in Form eines Bildleiters 5 untergebracht, das aus einem geordneten Glasfaserbündel besteht. Damit wird das am distalen Ende durch eine Linse aufgenommene Bild der zu untersuchenden Körperpartie zum Kopf 1 übertragen, wo es auf einen CCD-Sensor 6 eines Videokamera-Systems trifft. Das aufgenommene Bild ist damit auf einem Monitor zur gleichzeitigen Betrachtung durch mehrere Personen darstellbar.

Zur Erzeugung eines Lichtpunktes in der zu untersuchenden Körperpartie ist eine lasergestützte Lichtzeigereinrichtung 7 in das Endoskop integriert, die eine am Kopf 1 angeordnete Laserdiode 8 und eine davon ausgehende, durch die Röhre 4 zum distalen Ende hin geführte Lichtleitfaser 9 umfasst. An deren distalen Ende ist - falls notwendig - ein Linsensystem zur Fokussierung des auf der Lichtleitfaser austretenden Strahles angebracht. Die Laserdiode 8 ist durch einen nicht näher dargestellten Betätigungsknopf am Kopf 1 ein- und ausschaltbar.

Bei der in Fig. 1 und 2 gezeigten Version des Endoskops ist in der Röhre 4 konzentrisch zu der Längsachse der Bildleiter 5 und um diesen herum als zylindrische Anordnung das Lichtleitsystem 3 vorgesehen. Die Lichtleitfaser 9 ist dabei innerhalb des Lichtleitsystems 3 vom Kopf 1 zum distalen Ende des Endoskops geführt.

Bei der in Fig. 3 gezeigten Ausführungsform ist in der Röhre 4 eine nicht näher ausgeführte Tragstruktur 10 eingebracht, in der getrennt voneinander der Bildleiter 5, das Lichtleitsystem in Form zweier Glasfaserbündel und die Lichtleitfaser 9 laufen. Zusätzlich in ein Instrumenten- oder Spülkanal 11 in der Tragstruktur 10 vorgesehen.

## Patentansprüche

1. Endoskopisches oder minimalinvasives Instrument umfassend
- einen Kopf (1) zur Handhabung des Instruments und zum Anschluss externer Komponenten,
- eine starre oder flexible Röhre (4) am Kopf (1) zum Einführen in den zu untersuchenden Körper,
- gegebenenfalls ein optisches Betrachtungssystem (5) im Rohr (4) zur optischen Erfassung der zu untersuchenden Körperpartie, und
- gegebenenfalls ein Lichtleitsystem (3) in der Röhre (4) zur Beleuchtung der zu untersuchenden Körperpartie,
**gekennzeichnet durch**
- eine lasergestützte Lichtzeigereinrichtung (7) zur Erzeugung eines Leuchtpunktes in der zu untersuchenden oder zu behandelnden Körperpartie.

2. Instrument nach Anspruch 1, **dadurch gekennzeichnet, dass** die Lichtzeigereinrichtung eine durch die Röhre (4) verlaufende, am distalen Ende des Instruments ausmündende Lichtleitfaser (9) und einen in die Lichtleitfaser einstrahlenden Laser (8) am Kopf (1) des Instruments aufweist.

3. Instrument nach Anspruch 2, **dadurch gekennzeichnet, dass** der Laser ein durch eine Laserdiode (8) gebildet ist.

4. Instrument nach Anspruch 2 oder 3, **dadurch gekennzeichnet, dass** das optische Betrachtungssystem (5), das Lichtleitsystem (3) und die Lichtleitfaser (9) als getrennte Komponenten in die Röhre (1) eingebettet sind.

5. Instrument nach Anspruch 2 oder 3, **dadurch gekennzeichnet, dass das** optische System (5) und das Lichtleitsystem (3) als konzentrisch in der Röhre (1) ineinander sitzende Komponenten ausgeführt sind und die Lichtleitfaser (9) in das Lichtleitsystem (3) eingebettet ist.

6. Instrument nach einem der vorangegangenen Ansprüche, **dadurch gekennzeichnet, dass** die Lichtzeigereinrichtung (7) ein- und ausschaltbar ist.
